# EUROPÄISCHE PATENTSCHRIFT

(11) **EP 2 643 086 B1**
(45) Veröffentlichungstag und Bekanntmachung des Hinweises auf die Patenterteilung: **19.06.2019**
(21) Anmeldenummer: 11793371.3
(22) Anmeldetag: 22.11.2011
(51) Int. Cl.: B01J 35/02, C07C 51/215, C07C 51/25, C07C 67/05

(54) **KATALYSATORFORMKÖRPER FÜR DURCHSTRÖMTE FESTBETTREAKTOREN**
CATALYST SHAPED BODY FOR FLOW-CONDUCTING FIXED-BED REACTORS
CORPS MOULÉ DE CATALYSEUR POUR DES RÉACTEURS À LIT FIXE À ÉCOULEMENT CONTINU

(30) Priorität: 22.11.2010 DE 102010052126
(43) Veröffentlichungstag der Anmeldung: 02.10.2013
(73) Patentinhaber: Süd-Chemie IP GmbH & Co. KG, 81925 München (DE)
(72) Erfinder: REITZMANN, Andreas, 83022 Rosenheim (DE); BRANDSTÄDTER, Willi, Michael, 83607 Holzkirchen (DE); STREIFINGER, Leopold, 83052 Bruckmühl (DE); ESTENFELDER, Marvin, 85560 Ebersberg (DE)
(74) Vertreter: Kuba, Stefan
(86) Internationale Anmeldenummer: PCT/EP2011/070695
(87) Internationale Veröffentlichungsnummer: WO 2012/069481

(56) Entgegenhaltungen:
- WO-A1-2006/114320
- WO-A1-2007/051602
- WO-A2-2010/072721

## Beschreibung

Die vorliegende Erfindung betrifft einen Katalysatorformkörper zur katalytischen Umsetzung von organischen und anorganischen Verbindungen in Festbettreaktoren.

Maleinsäureanhydrid ist ein chemisches Zwischenprodukt von erheblichem gewerblichem Interesse. Es wird beispielsweise bei der Herstellung von Alkyd- und Polyesterharzen allein oder auch in Kombination mit anderen Säuren eingesetzt. Darüber hinaus stellt es auch ein vielseitig einsetzbares Zwischenprodukt für die chemische Synthese dar, zum Beispiel für die Synthese von Gamma-Butyrolacton, Tetrahydrofuran und 1,4-Butandiol, welche wiederum ihrerseits als Lösungsmittel eingesetzt werden oder zu Polymeren, wie beispielsweise Polytetrahydrofuran oder Polyvinylpyrrolidon, weiterverarbeitet werden.

Die Herstellung von Maleinsäureanhydrid (MSA) erfolgt in der Regel durch partielle Oxidation von Kohlenwasserstoffen in der Gasphase mit molekularem Sauerstoff oder mit einem molekularen Sauerstoff enthaltenden Gas in Gegenwart eines Vanadium-Phosphor-Oxid-Katalysators (VPO). Dabei werden verschiedene Oxidationskatalysatoren, verschiedene Katalysatorformkörper und verschiedene Verfahrensführungen angewandt. Im Allgemeinen enthalten die Oxidationskatalysatoren gemischte Oxide von Vanadium und Phosphor, wobei sich derartige Oxidationskatalysatoren mit Vanadium in einer Wertigkeit von +3,8 bis +4,8 als besonders geeignet für die Herstellung von Maleinsäureanhydrid aus gesättigten Kohlenwasserstoffen mit mindestens vier Kohlenstoffatomen in einer geraden Kette erwiesen haben. Neben Vanadium, Phosphor und Sauerstoff können die VPO-Katalysatoren auch Promotoren wie beispielsweise Metalle enthalten, die im Oxidationskatalysator in Form ihrer Oxide vorliegen können.

Zur Herstellung von z.B. Maleinsäureanhydrid durch heterogenkatalytische Gasphasenoxidation von Kohlenwasserstoffen werden Vanadium, Phosphor und Sauerstoff enthaltende Katalysatorformkörper mit einer voneinander verschiedenen Geometrie eingesetzt.

Die EP 1261424 B1 betrifft einen Katalysator für die Herstellung von Maleinsäureanhydrid durch heterogenkatalytische Gasphasenoxidation eines Kohlenwasserstoffs mit mindestens 4 Kohlenstoffatomen. Dieser Katalysator enthält eine katalytisch aktive Masse eines Vanadium-Phosphor-Mischoxids und weist eine im Wesentlichen hohlzylinderförmige Struktur auf. Dabei ist der Hohlzylinder derart ausgebildet, dass das Verhältnis der Höhe zum Durchmesser der Durchgangsöffnung höchstens 1,5 beträgt und das Verhältnis der geometrischen Oberfläche zum geometrischen Volumen des Formkörpers mindestens 2 mm⁻¹ beträgt.

Die EP 0552287 B1 beschreibt einen Katalysatorformkörper zur Herstellung von Maleinsäureanhydrid, wobei der Formkörper eine massive geometrische Form mit mindestens einem in der äußeren Oberfläche angeordneten Hohlraum umfasst. Der Formkörper ist dabei aus gemischten Oxiden von Vanadium und Phosphor gebildet und weist ein geometrisches Volumen von 30 % bis 67 % des geometrischen Volumens auf, das der Hohlraum freie massive Formkörper einnimmt, wobei das Verhältnis der geometrischen Oberfläche des Formkörpers zum geometrischen Volumen des Formkörpers mindestens 20 cm⁻¹ beträgt.

WO 2007051602 A1 betrifft einen Katalysatorformkörper zur Herstellung von Maleinsäureanhydrid, enthaltend gemischte Oxide des Vanadiums und des Phosphors als Katalysatorkomponente. Um einen gattungsgemäßen Katalysatorformkörper derart weiterzubilden, dass er verbesserte Eigenschaften aufweist, wird vorgeschlagen, dass der den Katalysatorformkörper umhüllende geometrische Grundkörper ein Prisma mit einer ersten und einer zweiten Dreiecksfläche ist und der Katalysatorformkörper mit drei durchgehenden Öffnungen versehen ist, die sich von einer ersten Fläche des Formkörpers, welche die erste Dreiecksfläche des Prismas aufspannt, zu einer zweiten Fläche des Formkörpers, welche die zweite Dreiecksfläche des Prismas aufspannt, erstrecken.

WO 0158590 A1 betrifft einen Pd/Ag-Trägerkatalysator, wobei der Formkörper eine trilobale Geometrie aufweist.

DE 10011307 A1 beansprucht einen VPO-Hohlzylinder zur Herstellung von MSA mit besonderen geometrischen Abmessungen, die im Prinzip auf relativ flache Hohlzylinder mit vergleichsweise großem innerem Loch hinauslaufen.

In DE 10211447 A1 wird ein VPO-Katalysator für die selektive Oxidation von Kohlenwasserstoffen mit mindestens 4 C-Atomen beansprucht, der im Wesentlichen eine hohlzylinderförmige Struktur aufweist und dessen Tablettendichte unterhalb eines nach einer empirischen Formel bestimmten Wertes liegt.

In EP 1127618 A1 werden geformte Katalysatoren in Form von Hohlzylindern offenbart, bei denen die Stirnflächen sowohl zum Außenrand als auch zum Rand der Innenbohrung hin abgerundet sind.

In EP 0004079 ist ein Katalysator für die Herstellung von Vinylacetat in der Gasphase beschrieben, der Edelmetalle der 8. Nebengruppe, Gold-, Alkali-, Erdalkali- und Cadmiumverbindungen auf einem Träger enthält, der einen sternförmigen Querschnitt aufweist.

DE3141942 C2 betrifft einen zylinderförmigen Katalysatorformkörper, der die Gestalt eines Kreiszylinders mit mehreren Längsvertiefungen besitzt, die eine bestimmte Tiefe und Breite aufweisen.

EP 220933 A1 beschreibt vierblattkleeförmige, katalytische Extrudate für Umsetzungen von Kohlenwasserstoffen in Festbettreaktoren (Quadrulobes).

In EP 1108470 B1 werden Katalysatoren mit einer aktiven Katalysatormasse auf inertem Träger in Form von Ringen beschrieben, die einen oder mehrere Kerben an der oberen und/oder unteren Flachseite des Ringes besitzen.

WO 2009121626 A1 betrifft ein Verfahren zur Herstellung eines nanokristallinen Molybdänmischoxids, und die Verwendung des Molybdänmischoxids in einem Katalysator für chemische Umsetzungen, z.B. in einem geträgerten Katalysator zur Umsetzung von Acrolein zu Acrylsäure.

WO 2010072721 A2 offenbart die katalytisch aktive Masse eines Katalysator-Formkörpers, die ein Vanadium und Phosphor enthaltendes Multielementoxid umfasst. Das spezifische Porenvolumen PV (in ml/g) des Katalysator-Formkörpers, die Schüttdichte p des Katalysator-Formkörpers (in kg/l), die geometrische Oberfläche A_{geo} (in mm²) und das geometrische Volumen V_{geo} (in mm³) des Katalysator-Formkörpers genügen der Bedingung: 0,275 < PV ^{∗} p ^{∗} A_{geo}/V_{geo}. In einem Verfahren zur Herstellung von Maleinsäureanhydrid durch heterogen katalytische Gasphasenoxidation eines Kohlenwasserstoffes erlaubt der Katalysatorformkörper einen geringeren Druckverlust und eine höhere Ausbeute. WO 2006114320 A1 betrifft einen zylinderförmigen Katalysatorkörper, der dadurch gekennzeichnet ist, dass auf der Umfangsfläche des Katalysatorkörpers Einprägungen vorgesehen sind.

Diese Einprägungen sind vorzugsweise als Rillen und Stege ausgestaltet, die parallel zur Längsachse des Katalysatorkörpers verlaufen. Dieser Katalysatorkörper eignet sich insbesondere zur Dampfreformierung von Kohlenwasserstoffen.

Aufgabe der vorliegenden Erfindung ist es, einen Katalysatorformkörper zur katalytischen Umsetzung von organischen und anorganischen Verbindungen zu schaffen, welcher im Vergleich zum Stand der Technik verbessert ist und insbesondere katalytische Umsetzungen von organischen und anorganischen Verbindungen mit einer höheren Selektivität und mit einer höheren Produktivität erlaubt.

Schüttungen aus Katalysatorformkörpern führen typischerweise bei Durchströmung mit einem Fluid (Gas/ Flüssigkeit) zu einen Druckverlust. Dadurch muss in fluiddurchströmten Festbettreaktoren ein gewisser Reaktoreingangsdruck aufgebracht werden, um die Reaktionsmischung durch Festbettreaktoren strömen zu lassen. Je höher der Druckverlust der Schüttung ist, desto höher muss der Reaktoreingangsdruck gewählt werden. Dies bedeutet, dass eine höhere Kompressorleistung aufgewendet werden muss, um das Fluid durch die Schüttung strömen zu lassen. Höhere Kompressorleistung bedeutet eine Erhöhung der Betriebskosten. Daher ist die Entwicklung von Formkörpern, die einen niedrigen Druckverlust aufweisen, sehr wünschenswert.

Gemäß der Erfindung wird ein Katalysatorformkörper zur katalytischen Umsetzung von organischen und anorganischen Verbindungen in Festbettreaktoren bereitgestellt, wobei der Katalysatorformkörper als Zylinder mit einer Außen-Grundfläche, einer Zylinderfläche, einer Zylinderachse und mindestens einer durchgehenden, parallel zur Zylinderachse verlaufenden Öffnung ausgebildet ist und die Außen-Grundfläche des Zylinders mindestens vier Loben aufweist, wobei ein den Katalysatorformkörper umschließender geometrischer Grundkörper ein Prisma ist, das eine Prismengrundfläche mit einer Länge und einer Breite aufweist, wobei die Länge größer ist als die Breite, wobei die Loben von Prismenecken der Prismengrundfläche umschlossen sind. Die Loben können als Abstandshalter zu benachbarten Formkörpern dienen.

Der Katalysatorformkörper gemäß hier beschriebener Ausführungsformen, hier auch Formkörper genannt, besitzt eine spezielle Geometrie, z.B. für Vollkatalysatoren, die, wenn eine Mehrzahl der Katalysatorformkörper als Schüttung in Rohrreaktoren eingebracht sind, zu einer Minimierung des Druckverlustes bei Durchströmung des Reaktors führen. Durch den geringeren Druckverlust sind mit Schüttungen aus den Katalysatorformkörpern gemäß Ausführungsformen bei gleichem Reaktoreingangsdruck höhere Strömungsgeschwindigkeiten und Raumgeschwindigkeiten (GHSV = Gesamtvolumenstrom/ Reaktorvolumen) realisierbar als mit Schüttungen aus Körpern mit höherem Druckverlust. Dadurch sind höhere Raum-Zeit-Ausbeuten (Volumenstrom Produkt/Reaktorvolumen) und Katalysatorproduktivitäten (Massenstrom Produkt/ Katalysatormasse) an Wertprodukt erzielbar. Pro Energieaufwand (Druckenergie) kann unter Verwendung der Katalysatorformkörper von Ausführungsformen mehr Wertprodukt erzeugt werden als mit Ringen. Im Vergleich zu Ringen bzw. Hohlzylindern weisen die Katalysatorformkörper hier beschriebener Ausführungsformen überraschenderweise auch eine höhere mechanische Stabilität auf. Die Anwendung der erfindungsgemäßen Katalysatorformkörper, hier auch Formkörper genannt, wird im Folgenden am Beispiel der partiellen Oxidation von Butan zu Maleinsäureanhydrid in der Gasphase in Festbettreaktoren beschrieben, ist aber nicht darauf beschränkt. Die Formkörper können in allen durchströmten Festbettreaktoren eingesetzt werden, die Schüttungen aus Katalysatorformkörpern enthalten und für die katalysierte Umsetzung von organischen und anorganischen Komponenten in Gas und Flüssigphase verwendet werden.

Die Katalysatorformkörper hier beschriebener Ausführungsformen zeichnen sich durch eine erhöhte spezifische Aktivität pro g/Katalysator und eine erhöhte Selektivität aus. Dadurch kann wiederum eine erhöhte Produktivität erhalten werden. Der Begriff "Produktivität" bedeutet den Massenstrom an beispielsweise MSA pro Katalysatormasse. Eine erhöhte Produktivität bedeutet, dass in einer bestehenden Produktionsanlage pro Zeiteinheit mehr Produkt, z. B. Maleinsäureanhydrid (MSA), synthetisiert werden kann.

Außerdem können für einen gegebenen maximalen Druckverlust einer Katalysatorschüttung nun mindestens 20% höhere Raumgeschwindigkeiten (GHSV = Volumenstrom/Katalysatorvolumen) angewandt werden, verglichen mit bekannten Formkörpergeometrien, wie z. B. Kugeln, massiven zylinderförmigen Tabletten oder Extrudaten. Ist z. B. mit einem der bisher bekannten Formkörpern maximal eine GHSV von 2500 h⁻¹ möglich, so sind unter Verwendung der Katalysatorformkörper gemäß Ausführungsformen Raumgeschwindigkeiten von mindestens 3000 h⁻¹ bei gleichem Druckverlust erzielbar. Aufgrund des spezifisch geringeren Druckaufbaus ist es andererseits auch möglich, einen gegebenen Durchsatz, z. B. GHSV von 2500 h⁻¹ mit einem geringeren Druckverlust als mit herkömmlichen Formkörpern zu realisieren. Dadurch muss eine geringere Gebläseleistung aufgewendet werden, was zu einer Einsparung von Energiekosten führt.

Ferner ermöglichen die Formkörper gemäß Ausführungsformen überraschenderweise eine Formgebung mit geringeren Press-und/oder Ausstoßkräften als z.B. ringförmige Formkörper. Dies vermindert den mechanischen Verschleiß der Werkzeuge, die zur Formung der Formkörper verwendet werden. Zur Erzeugung von Formkörpern eingesetzte Werkzeuge, beispielsweise Tablettierwerkzeuge mit Stempeln und Matrizen, werden stark mechanisch belastet. Je höher die mechanische Belastung ist, desto stärker werden die Werkzeuge verschlissen, was zu einer Verkürzung der Werkzeuglebenszeit führt. Diese unerwünschten Effekte können mit erfindungsgemäßen Formkörpern vermieden werden.

Weiterhin ist überraschenderweise die Maximaltemperatur im Katalysatorbett (Hot Spot Temperatur) mit Katalysatorformkörpern von Ausführungsformen geringer als bei bekannten Katalysatorformkörpern. Dies kann darauf zurückgeführt werden, dass durch die Geometrie erfindungsgemäßer Katalysatorformkörper in Katalysatorbetten eine verbesserte Wärmeabfuhr und Wärmeverteilung erreicht wird.

Ferner weisen die Katalysatorformkörper hier beschriebener Ausführungsformen eine hohe mechanische Stabilität auf, so dass beispielsweise beim Transport der Formkörper und Befüllen eines Rohrbündelreaktors mit den Katalysatorformkörpern eine Beschädigung der Formkörper im Wesentlichen ausbleibt. Darüber hinaus besitzen Katalysatorformkörper gemäß einiger Ausführungsformen abgerundete Begrenzungslinien. Dadurch wird ein einfacher und reproduzierbarer Befüllvorgang eines Reaktors ermöglicht mit einer geringen Ausbildung von Fülllücken.

Zudem besitzen Katalysatorformkörper gemäß Ausführungsformen verhältnismäßig kurze Diffusionswege. Die kurzen Diffusionswege bewirken einen hohen Porennutzungsgrad, so dass eine geringere Katalysatormasse zur Erzielung eines gewünschten Kohlenwasserstoffumsatzes eingesetzt werden kann sowie eine höhere Selektivität beispielsweise von MSA, da die Totaloxidation von MSA zu CO und CO₂ unterdrückt wird.

Ferner besitzen Katalysatorformkörper gemäß Ausführungsformen geringere Dichten bei mindestens gleicher Formkörperstabilität. Dies resultiert in höheren Porenvolumen und größeren Porendimensionen, was die Diffusion der Reaktanden und Produkte im Katalysatorkörper beschleunigt. Die verbesserte Diffusion führt zu höheren Porennutzungsgraden, so dass eine geringere Katalysatormasse zur Erzielung eines gewünschten Kohlenwasserstoffumsatzes eingesetzt werden kann sowie zu einer höheren Selektivität beispielsweise von MSA, da die Totaloxidation von MSA zu CO und CO₂ unterdrückt wird.

Bei dem erfindungsgemäßen Katalysatorformkörper ist der umhüllende geometrische Grundkörper ein Prisma, das eine Prismengrundfläche mit einer Länge und einer Breite aufweist, wobei die Länge größer ist als die Breite. Beispielsweise kann das Prisma ein Quader sein.

In einer bevorzugten Ausführungsform kann bei dem Katalysatorformkörper zwischen zwei benachbarten Loben eine Vertiefung in der Zylinderfläche vorgesehen sein. Alternativ oder zusätzlich kann zwischen zwei benachbarten Loben eine Erhebung in der Zylinderfläche vorgesehen sein. Der Katalysatorformkörper kann zwei entgegengesetzt angeordneten Vertiefungen und/oder zwei entgegengesetzt angeordneten Erhebungen umfassen. Die Erhebungen können auch als weitere Loben des Katalysatorformkörpers verstanden werden.

Der erfindungsgemäße Katalysatorformkörper weist eine parallel zur Zylinderachse verlaufende Öffnung auf. In einer weiteren Ausführungsform besitzt der Katalysatorformkörper vier Loben.

Bei den hier beschriebenen Ausführungsformen des Katalysator-formkörpers kann mindestens ein Element ausgewählt aus den Loben, der Vertiefung, den Vertiefungen, der Erhebung und den Erhebungen abgerundet sein. Die Loben, die Vertiefung(en) und die Erhebung(en) sind in diesem Fall von Kreisbögen begrenzt. Abgerundete Ecken des Katalysatorformkörpers werden hier auch Loben genannt. Bekannte prismatisch ausgebildete Katalysatorformkörper weisen entlang ihrer Längskanten in der Regel eine verhältnismäßig geringe Stabilität auf, so dass es beispielsweise beim Befüllvorgang eines Reaktors mit den entsprechenden Katalysatorformkörpern zu Abplatzungen im Bereich der Längskanten kommen kann. Dies wird durch die abgerundeten Elemente des Katalysatorformkörpers hier beschriebener Ausführungsformen vermieden.

Entsprechend einer fertigungstechnisch einfach zu realisierenden und damit kostengünstigen Ausführungsform des Katalysator-formkörpers weisen die durchgehenden Öffnungen einen kreisförmigen oder ovalen Querschnitt auf.

Bei einer Ausführungsform sind die Loben des Katalysatorformkörpers von Prismenecken der Prismengrundfläche des den Katalysatorformkörper umhüllenden geometrischen Grundkörpers umhüllt. Dabei können zwei Loben die Länge der Prismengrundfläche definieren und/oder zwei Loben die Breite der Prismengrundfläche definieren. Gemäß einer weiteren Ausführungsform können die Erhebung oder Erhebungen zwischen Loben vorgesehen sein, die die Länge definieren, und/oder die Vertiefung oder Vertiefungen zwischen Loben vorgesehen sein, die die Breite definieren.

Beispielsweise bei der Herstellung von Maleinsäureanhydrid durch heterogenkatalytische Gasphasenoxidation von Kohlenwasserstoffen treten im Reaktorbett Druckverluste auf, welche sich nachteilig auf den Gasdurchsatz und damit auf die Produktkapazität auswirken bzw. eine erhöhte Leistung des Gebläses erfordern. Um den Druckverlust im Reaktor möglichst gering zu halten und um möglichst kurze Diffusionswege innerhalb des Katalysatorformkörpers zu erzielen, weisen die durchgehenden Öffnungen des Katalysatorformkörpers gemäß einer besonders bevorzugten Ausführungsform einen Durchmesser von ungefähr 0,5 mm bis 3 mm, bevorzugt 1 mm bis 2,5 mm auf.

Um die Strömung des bei der Herstellung von Maleinsäureanhydrid durch heterogenkatalytische Gasphasenoxidation des durch das Katalysatorbett tretenden Gasgemisches positiv zu beeinflussen, d.h. den Diffusionsweg bei gleichzeitig ausreichender Stabilität zu verkürzen, kann fertigungstechnisch bevorzugt vorgesehen werden, dass die durchgehenden Öffnungen den gleichen Durchmesser aufweisen. Gemäß einer alternativen Ausführungsform kann vorgesehen sein, dass die durchgehenden Öffnungen einen voneinander verschiedenen Durchmesser aufweisen.

Ein Faktor, der die Fülldichte von Katalysatorformkörpern in einem Reaktor mitbestimmt, ist die Geometrie der Katalysatorformkörper. Um Einfluss auf die Fülldichte und um so Einfluss auf die Raumgeschwindigkeiten des durch das Katalysatorbett hindurchtretenden Gases zu nehmen, kann es gemäß einer weiteren Ausführungsform des Katalysatorformkörpers vorgesehen sein, dass mindestens zwei der mindestens vier abgerundeten Ecken, d.h. der Loben, den gleichen Außendurchmesser aufweisen. Gemäß einer alternativen Ausführungsform weisen mindestens zwei oder alle Loben den gleichen oder einen unterschiedlichen Außendurchmesser auf.

Ferner hängt die Fülldichte eines mit Katalysatorformkörpern beladenen Reaktors von der Größe der entsprechenden Formkörper ab. Um geeignete Raumgeschwindigkeiten des Kohlenwasserstoff und Sauerstoff enthaltenden Gasgemisches bei der Herstellung von Maleinsäureanhydrid durch heterogenkatalytische Gasphasenoxidation zu erhalten, weisen die Formkörper bevorzugt eine Höhe von ungefähr 2 bis 20 mm, insbesondere von 3 bis 10 mm, bevorzugt 3 bis 6 mm auf. Die Höhe ist die Abmessung des Katalysatorformkörpers parallel zur Zylinderachse.

Gemäß einer weiteren Ausführungsform sind mindestens zwei der Loben voneinander verschieden. Dabei kann der Schwerpunkt jeweils von der Zylinderachse ungefähr 1,5 bis 5 mm, bevorzugt 2,5 bis 4 mm, entfernt sein. Die abgerundeten Ecken können einen Durchmesser der Rundung im Bereich von ungefähr 1 bis 2 mm besitzen. Der Radius des von den abgerundeten Ecken jeweils gebildeten Kreisbogens kann im Bereich von ungefähr 0,5 bis 2,5 mm liegen. Der Radius des von den abgerundeten Erhebungen jeweils gebildeten Kreisbogens kann im Bereich von ungefähr 2 bis 3,5 mm liegen. Ferner kann der Radius des von den abgerundeten Vertiefungen jeweils gebildeten Kreisbogens im Bereich von ungefähr 1 bis 9 mm liegen.

Bei einer anderen Ausführungsform können die Loben des Katalysatorformkörpers derart in der Außen-Grundfläche angeordnet sein, dass der Winkel zwischen der Senkrechten auf eine Gerade durch die Schwerpunkte zweier Loben, die die Breite der Prismengrundfläche definieren, und einer Geraden durch den Schwerpunkt dieser Loben und durch die Zylinderachse ungefähr 10° bis 75°, bevorzugt 25° bis 60°, beträgt.

Ferner kann in einer Ausführungsform des Katalysatorformkörpers der Außendurchmesser des Zylinders oder der Abstand zwischen zwei gegenüberliegenden Vertiefungen ungefähr 3 bis 10 mm, bevorzugt 5 bis 8 mm, betragen. Der Durchmesser der Öffnung oder Öffnungen des Katalysatorformkörpers kann 0,5 bis 4 mm, bevorzugt 1 bis 3 mm betragen.

Wie oben erläutert, ist in Beispielen von Ausführungsformen der umhüllende geometrische Grundkörper ein Quader. In weiteren Ausführungsformen kann das Verhältnis des Volumens des erfindungsgemäßen Formkörpers zum Volumen des umgebenden Quaders größer als 80 % ohne Berücksichtigung der Öffnung(en), d.h. einschließlich des Volumens der Öffnung(en), und größer 70 % unter Berücksichtigung der Öffnung(en) sein, d.h. ausschließlich des Volumens der Öffnung(en).

Der erfindungsgemäße Formkörper hat in Ausführungsformen eine geometrische Oberfläche von ungefähr 0,15 cm² bis 5 cm², vorzugsweise 0,5 cm² bis 4 cm², besonders bevorzugt 1 cm² bis 3,5 cm², insbesondere 1,5 cm² bis 3 cm².

Gemäß einer weiter bevorzugten Ausführungsform des Katalysatorformkörpers beträgt das Verhältnis der geometrischen Oberfläche des Formkörpers zum Volumen des Formkörpers ungefähr 1 bis 1,8 mm⁻¹, und vorzugsweise ist das Verhältnis der geometrischen Oberfläche des Formkörpers zu seinem Volumen mindestens 1,2 mm⁻¹ oder zwischen 1,2 und 1,8 mm⁻¹, wobei als Volumen des Formkörpers das Volumen abzüglich des Volumens der Öffnung(en) herangezogen wurde, d.h. unter Berücksichtigung der Öffnung(en).

Entsprechend einer Ausführungsform des Katalysatorformkörpers ist die Schüttdichte des Katalysatorformkörpers kleiner als 0,75 kg/l, bevorzugt zwischen 0,45 und 0,7 kg/l.

Die Herstellung von Maleinsäureanhydrid durch heterogenkatalytische Gasphasenoxidation als ein Beispiel wird in der Regel in sogenannten Rohrbündelreaktoren durchgeführt, in welchen in senkrecht ausgerichteten Rohren Katalysatorformkörper übereinandergeschichtet sind. Entsprechend muss ein Katalysatorformkörper dem Gewicht der über ihn liegenden Formkörper widerstehen können. Gemäß einer weiter bevorzugten Ausführungsform des Formkörpers ist eine mittlere unidirektionale Seitendruckfestigkeit größer als 28 N, bevorzugt zwischen 30 und 60 N, und/oder eine andere mittlere unidirektionale Seitendruckfestigkeit, d.h. die Seitendruckfestigkeit in einer anderen Richtung, größer als 70 N, bevorzugt zwischen 80 und 240 N.

Die BET-Oberfläche des erfindungsgemäßen Katalysatorformkörpers kann ungefähr 10 bis 300 m²/g, vorzugsweise 12 bis 80 m²/g, besonders bevorzugt 15 - 50 m²/g betragen. Die BET-Oberfläche wird nach der Einpunkt-Methode durch Adsorption von Stickstoff nach DIN 66132 bestimmt.

Weiter kann es bevorzugt sein, dass das integrale Porenvolumen (bestimmt gemäß DIN 66133 (Hg-Porosimetrie)) > 100, bevorzugt > 180 mm³/g beträgt.

Die Katalysatorformkörper gemäß Ausführungsformen können die katalytisch aktiven Komponente(n), hier auch Katalysatorkomponente(n) genannt, beispielsweise in reiner, unverdünnter Form als sogenannter "Vollkatalysator" oder verdünnt mit einem bevorzugt oxidischen Trägermaterial als Trägerkatalysator oder als sogenannter geträgerter "Katalysator" enthalten.

Typischerweise werden in der sog. heterogenen Katalyse zwei Arten von (Feststoff)Katalysatoren unterschieden (J. Weitkamp und R. Gläser in: Winnacker/Küchler "Chemische Technik: Prozesse und Produkte", Band 1, Kap. 5, Wiley-VCH, 2004):
Zum einen gibt es sogenannte "geträgerte Katalysatoren", auch Coat- oder Beschichtungskatalysatoren genannt, unter denen man Feststoff-Katalysatoren versteht, die durch Beschichten eines (typischerweise nicht porösen) Trägerkörpers mit einer porösen Schicht enthaltend die eigentlich katalytisch aktive Spezies hergestellt werden.

Im Unterschied dazu wird bei den sog. "Trägerkatalysatoren" durch Imprägnierverfahren die katalytisch aktive Spezies, z.B. Edelmetalle, wie Pd, Pt, Au, Ag etc., als Lösung einer (reduzierbaren) Verbindung dieser Spezies dispers auf einen porösen Träger, wie z.B. SiO₂, Al₂O₃, TiO₂, ZrO₂, etc. aufgebracht. Bei den durch das Imprägnierverfahren hergestellten Trägerkatalysatoren bestehen zumeist chemisch-physikalische Wechselwirkungen zwischen Träger und aktiven Spezies, die entscheidenden Einfluss auf das katalytische Geschehen nehmen.

Bei den geträgerten Katalysatoren dient der Trägerkörper lediglich der Formgebung ("structural support"). Im Gegensatz zu Trägerkatalysatoren, bei denen die aktiven Elemente dispers in dem porösen Träger - gegebenenfalls auch in einer auf dem Träger angeordneten äußeren Schale (= Schalenkatalysator) - verteilt sind, ist beim geträgerten Katalysator der typischerweise nicht poröse Trägerkörper von einer die aktive Spezies enthaltenden Schicht umhüllt.

Geeignete Trägermaterialien für die geträgerten Katalysatoren sind beispielsweise Aluminiumoxid, Siliziumdioxid, Aluminiumsilikate, Zirkondioxid, Titandioxid oder Gemische davon. Vorzugsweise beträgt der Gehalt der Katalysatorkomponente im Katalysatorformkörper ungefähr 3 bis 50 Gew.-% bezogen auf das Gesamtgewicht des Katalysatorformkörpers. Im Falle eines geträgerten Katalysators beträgt der Gehalt der Katalysatorkomponente im Katalysatorformkörper 3 - 50 Gew.-%, bevorzugt 5 - 30 Gew.-% bezogen auf das Gesamtgewicht des Katalysatorformkörpers.

Der Katalysatorformkörper von Ausführungsformen kann als Vollkatalysator, als geträgerter Katalysator oder als Trägerkatalysator ausgebildet sein. Als katalytisch aktive Komponente kann der Katalysatorformkörper Oxide des Vanadiums und des Phosphors umfassen, z.B. für die Herstellung von Maleinsäureanhydrid aus n-Butan. Andere katalytisch aktive Komponenten des Katalysatorformkörpers können ein oder mehrere Metalle der Nebengruppen des Periodensystems, ein oder mehrere Metalloxide oder Metallmischoxide von Metallen der Nebengruppen des Periodensystems sein. Z.B. kann der Formkörper Bi, Mo, Fe, Ni, W Sb, Co, Mg, Zn, Si, K, Cs, deren Oxide und/oder deren Mischoxide als katalytische aktive Komponenten umfassen, beispielsweise für die Umsetzung von Propen zu Acrolein. Der Formkörper kann auch Mo, V, W, Cu, Sb, deren Oxide und/oder deren Mischoxide als katalytisch aktive Komponenten umfassen, beispielsweise für die Umsetzung von Acrolein zu Acrylsäure. Auch kann der Formkörper z.B. Mo, V, Te, Nb, Sb, enthalten für z.B. die Umsetzung von Propan zu Acrylsäure. Auch ein oder mehrere Edelmetalle, wie Pd, Pt, Au und/oder Ag können als katalytisch aktive Komponenten kann der Formkörper von Ausführungsformen umfassen, z.B. für die Herstellung von Vinylacetat aus Ethen in Gegenwart von Essigsäure.

Der Katalysatorformkörper kann in Ausführungsformen als weitere katalytisch aktive Komponente einen Promotor, der aus Metallen des Periodensystems der Elemente ausgewählt ist, enthalten.

Gemäß einer bevorzugten Ausführungsform des Katalysatorformkörpers enthält die Katalysatorkomponente Oxide des Vanadiums und des Phosphors und entspricht der allgemeinen Formel

VPₓO_{y}M_{z}

worin M zumindest ein Promotor ist, x eine Zahl von 0,1 bis 3 darstellt, y eine den Wertigkeiten von V, P und M angepasste Zahl ist und z eine Zahl von 0 bis 1,5 darstellt.

Wie bereits vorstehend ausgeführt, kann der Promotor aus den Metallen ausgewählt sein. Vorzugsweise ist der Promotor ausgewählt aus Chrom, Nickel, Magnesium, Aluminium, Silizium, Wolfram, Niob, Antimon und/oder Cäsium.

Je nach Verfahrensführung kann es bevorzugt sein, noch andere Promotorelemente als die vorerwähnten einzusetzen. Bei entsprechender Verfahrensführung kann es daher bevorzugt sein, wenn der Promotor weiter ausgewählt ist aus Lithium, Zink, Eisen, Bismut, Tellurium, Silber, Molybdän und/oder Zirkonium.

Günstig ist es, wenn der Anteil des Promotors in Form eines Oxids oder in Form einer Verbindung, die in ein Oxid überführt werden kann, 0,005 Gew.-% bis 5 Gew.-% beträgt, bezogen auf das Gesamtgewicht des Formkörpers.

Dem Katalysatorformkörper können auch Hilfsmittel zugesetzt sein, wie beispielsweise Tablettierhilfsmittel oder Porenbildner. Tablettierhilfsmittel werden im Allgemeinen zugesetzt, wenn die Formgebung des Katalysatorformkörpers über eine Tablettierung erfolgt. Tablettierhilfsmittel sind in der Regel katalytisch inert und verbessern die Tablettierungseigenschaften des so genannten Katalysatorprecursorpulvers, beispielsweise durch Erhöhung der Gleit- und/oder Rieselfähigkeit. Ein besonders geeignetes Tablettierungshilfsmittel ist beispielsweise Graphit. Die zugesetzten Tablettierungshilfsmittel können im aktivierten Katalysator verbleiben und liegen in der Regel in einer Größenordnung von 1 bis 5 Gew.-% in dem Katalysatorformkörper vor bezogen auf das Gesamtgewicht des Katalysatorformkörpers.

Darüber hinaus kann der Katalysatorformkörper Porenbildner enthalten. Porenbildner sind Stoffe, welche zur gezielten Einstellung der Porenstruktur im Meso- und Makroporenbereich eingesetzt werden. Dabei handelt es sich in der Regel um Kohlenstoff, Wasserstoff, Sauerstoff und/oder Stickstoff enthaltende Verbindungen, welche vor der Formgebung dem Katalysatorprecursorpulver zugesetzt werden und sich bei der anschließenden Aktivierung des Katalysatorformkörpers beispielsweise durch Kalzinieren zersetzen oder verdampfen und somit zum überwiegenden Teil aus dem entstehenden Formkörper austreten und dabei Poren erzeugen.

Der erfindungsgemäße Katalysatorformkörper kann verwendet werden für eine Partialoxidationsreaktion, für eine Partialoxidationsreaktion eines oder mehrerer Kohlenwasserstoffe, zur Herstellung von Maleinsäureanhydrid aus Kohlenwasserstoff, zur Herstellung von Vinylacetatmonomer durch Oxidation von Ethen in Gegenwart von Essigsäure, oder zur Oxidation von Propen oder Propan zu Acrolein und/oder Acrylsäure. Eine Ausführungsform betrifft die Verwendung des Katalysatorformkörpers gemäß vorstehender Ausführungsformen für Partialoxidationsreaktionen, für Partialoxidationsreaktionen von Kohlenwasserstoffen oder zur Herstellung von Maleinsäureanhydrid aus Kohlenwasserstoffen. Bei einer derartigen Verwendung kann als Kohlenwasserstoff n-Butan eingesetzt werden. Im Folgenden wird eine Verwendung von Ausführungsformen zur Herstellung von Maleinsäureanhydrid aus Kohlenwasserstoffen beschrieben, ohne die Erfindung darauf einzuschränken.

Als Kohlenwasserstoffe können dabei nicht-aromatische Kohlenwasserstoffe mit 4 bis 10 Kohlenstoffatomen zum Einsatz kommen. Dabei ist es erforderlich, dass der Kohlenwasserstoff nicht weniger als 4 Kohlenstoffatome in einer geraden Kette oder in einem Ring enthält. Besonders geeignet ist der Kohlenwasserstoff n-Butan. Außer n-Butan sind auch Pentane, Hexane, Heptane, Octane, Nonane, Decane oder Gemische von beliebigen dieser Verbindungen mit oder ohne n-Butan geeignet, sofern sie mindestens 4 Kohlenstoffatome in gerader Kette enthalten.

Ungesättigte Kohlenwasserstoffe können ebenfalls für die Umwandlung in Maleinsäureanhydrid eingesetzt werden. Geeignete ungesättigte Kohlenwasserstoffe sind beispielsweise Butene (1-Buten und 2-Buten), 1,3-Butadien, die Pentene, die Hexene, die Heptene, die Octene, die Nonene, die Decene sowie Gemische beliebiger dieser Verbindungen mit der Maßgabe, dass sie mindestens 4 Kohlenstoffatome in gerader Kette enthalten. Ebenso geeignet sind substituierte und nicht substituierte Furane, z. B. Tetrahydrofuran, außerdem aromatische Verbindungen, beispielsweise Benzol und dessen Derivate.

Der Katalysatorformkörper gemäß hier beschriebener Ausführungsformen kann beispielsweise wie in der WO 97/12674 A1 beschrieben hergestellt werden, wobei die Formgebung entsprechend der Geometrie der Ausführungsformen erfolgt.

Die wesentlichen Schritte einer möglichen Herstellung eines VPO-Katalysatorformkörpers unter Ausbildung eines Katalysatorprecursorpulvers, Formgebung und anschließender Aktivierung sind im Folgenden kurz als ein Beispiel erläutert:
- Umsetzung einer fünfwertigen Vanadiumverbindung (beispielsweise V₂O₅) mit einem reduzierenden Lösungsmittel (beispielsweise Isobutanol) in Anwesenheit einer fünfwertigen Phosphorverbindung (beispielsweise o-Phosphorsäure oder einer anderen Phosphorsäure wie Pyrophosphorsäuren und/oder deren Gemische etc.) und ggf. eines Promotors. Die vorgenannte Reaktion kann ggf. in Gegenwart eines Trägermaterials durchgeführt werden, das beispielsweise pulverförmig vorliegt und in dem Lösungsmittel dispergiert wird.
- Gewinnung des gebildeten Vanadium-, Phosphor- und Sauerstoff-enthaltenden Katalysatorprecursors, beispielsweise mittels Filtration, Eindampfung, Dekantieren oder Zentrifugieren.
- Trocknung und ggf. Kalzinieren des Katalysatorprecursors. Dem getrockneten Katalysatorprecursor kann ggf. pulverförmiges Trägermaterial und/oder ein Porenbildner untergemischt werden. Die Trocknung kann beispielsweise im Vakuum unter Schutzgas oder unter Sauerstoffüberschuss erfolgen.
- Formgebung durch Überführung in die Geometrie gemäß hier beschriebener Ausführungsformen. Vor der Formgebung kann dem getrockneten Katalysatorprecursor ein Tablettierhilfsmittel zugesetzt werden.
- Aktivierung des Vanadium-, Phosphor- und Sauerstoff- und ggf. Promotor enthaltenden Katalysatorprecursors durch Erhitzen in einer Atmosphäre, welche Sauerstoff, Stickstoff, Edelgase, Kohlendioxid, Kohlenwasserstoffe, Kohlenmonoxid und/oder Wasserdampf bzw. Mischungen davon enthalten kann. Durch die Auswahl von Temperatur, Aufheizrate, Behandlungsdauer und Gasatmosphäre können die mechanischen und/oder katalytischen Eigenschaften des Katalysatorformkörpers bestimmt werden.

Der Katalysatorformkörper kann beispielsweise hergestellt werden, indem das getrocknete Katalysatorprecursorpulver zunächst mit einem Bindemittel oder mit einem Gleitmittel vermischt wird. Die Herstellung des Formkörpers erfolgt beispielsweise dann in einer Tablettenpresse mit einem Drehteller, an dessen Umfang mehrere Öffnungen mit einem entsprechenden Querschnitt, beispielsweise einem quadrulobalen Querschnitt, angeordnet sind. In diese Öffnung (Matrizen) wird die Mischung eingefüllt, die von unten von einem Stempel gehalten wird, durch den während der Drehung des Drehtellers bspw. drei Zapfen, die an den Stellen der zu erzeugenden Öffnungen liegen, nach oben geschoben werden. Bei der weiteren Drehung des Drehtellers greift von oben ein Stempel mit einem entsprechenden Querschnitt ein, der mit Öffnungen versehen ist, in welche die Zapfen beim Herunterdrücken des oberen Stempels eindringen. Die gepressten Formkörper werden bei der weiteren Drehung des Drehtellers nach dem Zurückziehen des unteren Stempels und dem Weiterschieben des oberen Stempels aus den Matrizen herausgedrückt. Der so entstandene Katalysatorformkörper wird danach aktiviert, z.B. durch Kalzinieren.

Weitere Merkmale und Zweckmäßigkeiten ergeben sich aus der folgenden Beschreibung von Ausführungsformen, den Figuren und den Unteransprüchen.

Alle hier beschriebenen und sich nicht gegenseitig ausschließenden Merkmale von Ausführungsformen können miteinander kombiniert werden. Gleiche Elemente der Ausführungsformen sind in der folgenden Beschreibung mit den gleichen Bezugszeichen versehen. Die Abmessungen und Winkel hier beschriebener Ausführungsformen und Beispiele können zuzüglich üblicher Mess- und Fertigungs-Toleranzen verstanden werden. Elemente einer Ausführungsform können in den anderen Ausführungsformen genutzt werden ohne weitere Erwähnung. Ausführungsformen der Erfindung werden nun durch die nachfolgenden Beispiele anhand von Figuren genauer beschrieben, ohne sie dadurch einschränken zu wollen. Es zeigen:
- Fig. 1a und 1b: einen Katalysatorformkörper gemäß eines ersten Beispiels;
- Fig. 2a und 2b: einen Katalysatorformkörper gemäß eines zweiten Beispiels;
- Fig. 3a und 3b: Katalysatorformkörper gemäß eines dritten und vierten Beispiels; und
- Fig. 4: das Druckverhalten der ersten bis dritten Beispiele im Vergleich zu einem Vergleichsbeispiel.

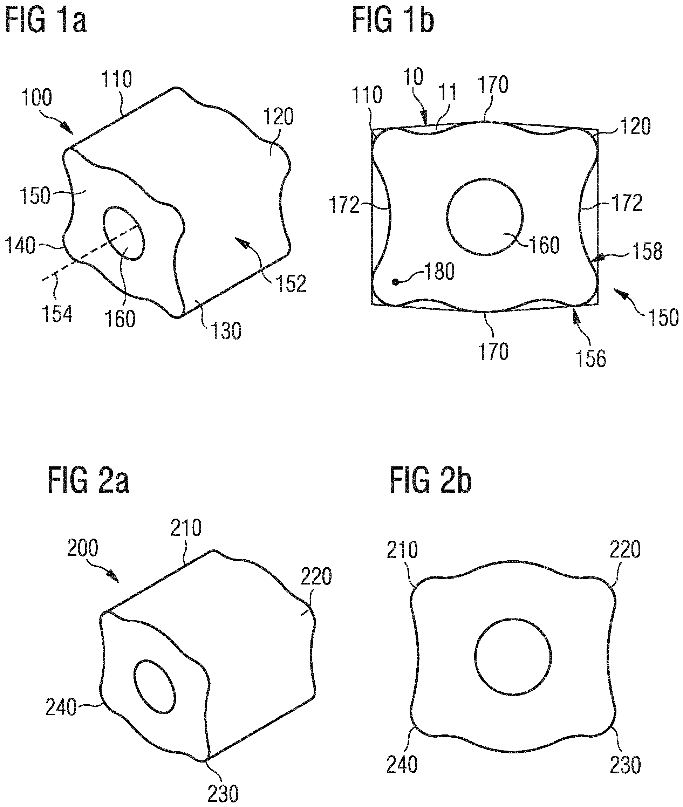

In Fig. 1a und 1b ist ein Katalysatorformkörper 100 gemäß eines ersten Beispiels (Beispiel 1) einer Ausführungsform gezeigt. Der Katalysatorformkörper ist als Zylinder mit einer Außen-Grundfläche 150, einer Zylinderfläche 152, einer Zylinderachse 154 und mindestens einer durchgehenden, parallel zur Zylinderachse verlaufenden Öffnung 160 ausgebildet. Ein den Katalysatorformkörper 100 umhüllender geometrischer Grundkörper ist ein Prisma 10 mit einer sechseckigen Grundfläche 11, wie in Fig. 1b dargestellt. Als ein alternativer, den Katalysatorformkörper umhüllender, geometrischer Formkörper kann ein Prisma mit einer viereckigen Grundfläche (nicht gezeigt) gewählt werden, z.B. ein Quader.

Fig. 1a zeigt eine perspektivische Ansicht des Katalysatorformkörpers 100, während Fig. 1b eine Draufsicht auf die Außen-Grundfläche 150 des Katalysatorformkörpers sowie auf die Grundfläche 11 des Prismas 10 darstellt. Der Zylinder wie auch die Außen-Grundfläche 150 des Zylinders besitzt vier abgerundete Ecken 110, 120, 130 und 140, d.h. Loben, die sich entlang des Zylinders parallel zur Zylinderachse erstrecken. Die Loben 110 und 120 bzw. 130 und 140 bilden miteinander jeweils eine lange Seite 156 der Außen-Grundfläche 150. Die Loben 120 und 130 bzw. 140 und 110 bilden miteinander jeweils eine breite Seite 158 der Außen-Grundfläche 150. Die Abmessungen der langen Seiten 156 sind größer als die Abmessungen der breiten Seiten 158. Die Abmessung der langen Seite 156 beträgt 7,2 mm, die Abmessung der breiten Seite 158 beträgt 5,5 mm.

Die vier Loben 110, 120, 130, 140 weisen jeweils den gleichen Außendurchmesser von 1,5 mm auf, d.h. der Radius des jeweils von den Loben gebildeten Kreisbogens beträgt 0,75 mm. Auf den langen Seiten 156 ist zwischen den Loben jeweils eine Erhebung 170 vorgesehen, während auf den breiten Seiten 158 zwischen den Loben Vertiefungen 172 vorhanden sind. Der Katalysatorformkörper 100 kann daher als Doppelalpha bezeichnet werden. Er ist als Ring bzw. Hohlzylinder ausgeführt, wobei die Loben als Abstandshalter dienen. Der Außendurchmesser der Zylinder, d.h. der Abstand zwischen den Vertiefungen 172 beträgt 6 mm. Der Durchmesser der Öffnung 160 beträgt 2,4 mm.

Die Loben 110 bis 140 besitzen jeweils einen Schwerpunkt 180, wobei der Schwerpunkt jeweils von der Zylinderachse 3,5 mm entfernt ist.

Die Position der abgerundeten Ecken oder Loben 110 bis 140 definiert sich über einen Winkel. Die Loben 110 bis 140 sind derart in der Außen-Grundfläche angeordnet, dass der Winkel zwischen der Senkrechten auf eine Gerade durch die Schwerpunkte der Loben 120 und 130 bzw. 140 und 110, die die Breite der Prismengrundfläche definieren, und einer Geraden durch den Schwerpunkt dieser Loben und durch die Zylinderachse 35° beträgt.

Die Länge der Loben definiert sich über den Abstand von der Zylinderachse zum Schwerpunkt der Loben, die in diesem Beispiel 3,5 mm beträgt.

Die Radien der von den Erhebungen 170 gebildeten Kreisbögen betragen jeweils 6 mm und die Radien der von den Vertiefungen 172 gebildeten Kreisbögen betragen jeweils 3 mm. Die Radien der zwischen den Loben 110 bis 140 und den Erhebungen 170 gebildeten Vertiefungen, d.h. deren Kreisbögen, betragen jeweils 1,8 mm. Die Erhebungen 170 können als zwei weitere Loben des Katalysatorformkörpers 100 verstanden werden, die von den Loben 110 bis 140 verschieden sind.

Die Höhe, d.h. die Länge parallel zur Zylinderachse 154, des Katalysatorformkörpers 100 beträgt 6 mm. Die einhüllende geometrische Form ist das Prisma 10, dessen Querschnitt länger als breit ist.

Die sechseckige Grundfläche des den Katalysatorformkörper 100 umhüllenden Prismas 10 wird im vorliegenden Beispiel durch die Loben 110 bis 140 und die Erhebungen 170 aufgespannt. Alternativ kann, wie oben erwähnt, im Beispiel 1 das den Formkörper 100 umhüllende Prisma ein Quader mit einer rechteckigen Grundfläche sein, deren kurze Seite von den Erhebungen 170 und deren lange Seite von den Loben 110 und 120 bzw. 130 und 140 definiert wird.

In Fig. 2a und 2b ist ein Katalysatorformkörper 200 gemäß eines zweiten Beispiels (Beispiel 2) einer Ausführungsform gezeigt. Der Formkörper 200 unterscheidet sich von dem Formkörper 100 in der Länge der Loben 210, 220, 230, 240, die in diesem Beispiel 3 mm beträgt.

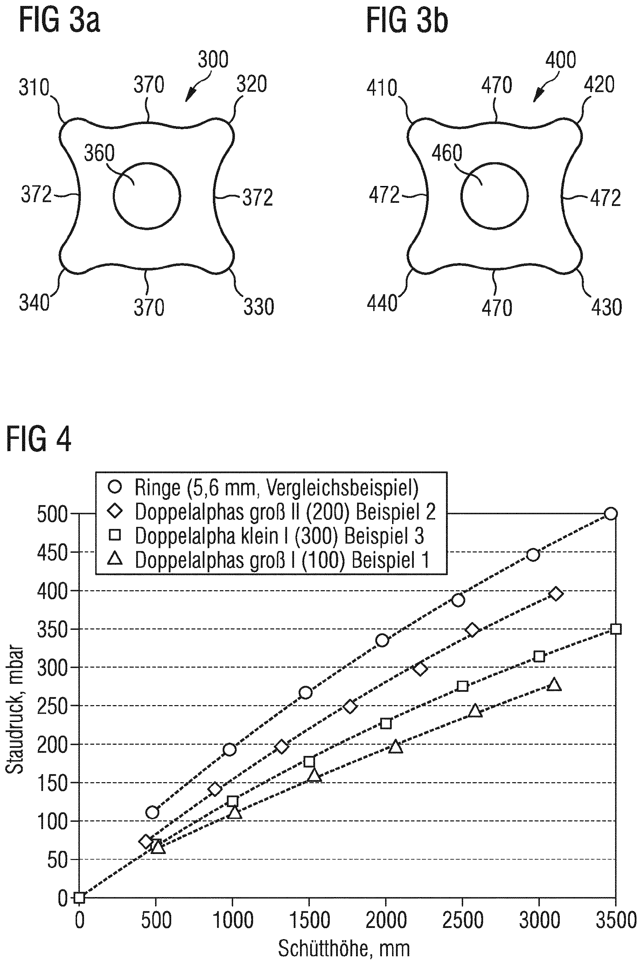

Fig. 3a und 3b veranschaulichen als ein drittes und ein viertes Beispiel (Beispiele 3 und 4) einer Ausführungsform die Katalysatorformkörper 300 und 400. Die Katalysatorformkörper 300 und 400 dieser Beispiele sind kleiner als die Formkörper 100 und 200 ausgeführt, mit folgenden Abmessungen: die Höhe, d.h. die Abmessung des Katalysatorformkörpers parallel zur Zylinderachse, beträgt 5,5 mm. Der Durchmesser der Öffnung 360 bzw. 460 beträgt 2,4 mm. Die Länge der Loben 310 bis 340 bzw. 410 bis 440 beträgt 3,5 mm. Die langen Seiten der Außen-Grundfläche haben eine Abmessung von 6,67 mm. Die breiten Seiten der Außen-Grundfläche besitzen eine Abmessung von 5,9 mm. Der Winkel zur Definition der Position der Loben beträgt jeweils 40°. Der Durchmesser der Loben 310 bis 340 und 410 bis 440 beträgt 1,4 mm. Die Vertiefungen 372 und 472 besitzen als Begrenzung Kreisbögen mit Radius 4 mm. Die Erhebungen 370 und 470 bilden Kreisbögen jeweils eines Radius von 2,75 mm. Die Radien der zwischen den Loben 310 bis 340 und den Erhebungen 370 bzw. 470 gebildeten Vertiefungen betragen für das dritte Beispiel, Formkörper 300 (Fig. 3a), 2,5 mm und für das vierte Beispiel, Formkörper 400 (Fig. 3b), 1,5 mm.

Im den Beispielen 3 und 4 ist das den jeweiligen Formkörper 300 bzw. 400 umhüllende Prisma ein Quader mit einer rechteckigen Grundfläche (nicht gezeigt), die von den vier Loben des jeweiligen Formkörpers aufgespannt wird.

Erfindungsgemäß kann der Radius des von den abgerundeten Loben 110 bis 140, 210 bis 240, 310 bis 340 und 410 bis 440 jeweils gebildeten Kreisbogens im Bereich von 0,5 bis 2,5 mm liegen. Der Radius des von den abgerundeten Erhebungen 170, 270, 370 und 470 jeweils gebildeten Kreisbogens kann erfindungsgemäß im Bereich von 2 bis 3,5 mm liegen. Ferner kann erfindungsgemäß der Radius des von den abgerundeten Vertiefungen 172, 272, 372 und 472 jeweils gebildeten Kreisbogens im Bereich von 1 bis 9 mm liegen.

Ein Vergleichsbeispiel besteht aus einem Ring mit einem Durchmesser von 5,6 mm, einer Abmessung (Höhe) entlang der Ringachse von 5,6 mm und eines Lochdurchmessers von 2,4 mm.

Die Beispiele 1 bis 3 sowie das Vergleichsbeispiel bestanden aus Vanadium-Phosphor-Mischoxid (VPO, molares Verhältnis P/V = 1,07; Kohlenstoffgehalt des Formkörpers 4,2 Gew.%; Oxidationszahl Vanadium VOₓ 4,2; BET Oberfläche des Formkörpers 22 m²/g, gemessen nach DIN 66131). Zusätzliche Beispiele 1a, 2a, 3a, und ein Vergleichsbeispiel a waren entsprechend den Formkörpern der Beispiele 1 bis 3 sowie des Vergleichsbeispiels geformt und bestanden aus einem Al₂O₃/SiO₂ Mischoxid (Al₂O/SiO₂ = 90/10, Hersteller: Sasol, BET Oberfläche: 400 m²/g, Porenvolumen: 0,75 ml/g). Weitere, hier nicht näher beschriebene Beispiele waren Formkörper aus SiO₂, TiO₂ und ZrO₂, die wie die Formkörper der Beispiele 1 bis 3 sowie des Vergleichsbeispiels geformt waren.

Die auftretenden Presskräfte während der Tablettierung der Beispiele 1 bis 3 und des Vergleichsbeispiels in einer Rundläufertablettenpresse RoTabT Fa. Kg-pharma sind in Tabelle 1 wieder gegeben. Die in Tabelle 1 aufgeführten Press- und Ausstoßkräfte wurden an der Tablettenpresse abgelesen.

**Tabelle 1**

| Probe | Ringe Vergleichsbeispiel | Doppelalphas groß I (100), Beispiel 1 | Doppelalphas groß II (200), Beispiel 2 | Doppelalphas klein I (300), Beispiel 3 |
|---|---|---|---|---|
| Presskraft, kN | 9,0-9,8 | 3,8 - 4,3 | 4,0 - 4,5 | 2,0 - 2,2 |
| Ausstoßkraft, N | 750 -800 | 340 - 380 | 350 - 400 | 280 -320 |

Wenn hohe Presskräfte aufgewendet werden müssen, um Katalysatorformkörper in Form von Tabletten mit geforderter Stabilität (Seitendruckfestigkeit) herstellen zu können, und hohe Ausstoßkräfte (= Reibung beim Auswurf der Tabletten) wirken, werden die Tablettierwerkzeuge, d.h. Stempel und Matrizen, stark mechanisch belastet. Je höher die mechanische Belastung ist, desto stärker werden die Werkzeuge verschlissen, was zu einer Verkürzung der Werkzeuglebenszeit führt. Bei den erfindungsgemäßen Formen treten geringere Press- und Ausstoßkräfte auf als bei den Ringen, insbesondere beim Formkörper 300.

Das verbesserte Druckverlustverhalten von Schüttungen der Formkörper 100, 200 und 300 im Vergleich zu einer Schüttung des Rings aus dem Vergleichsbeispiel zeigt sich in Fig. 4. Die Messungen wurden bei Raumtemperatur durchgeführt und der Volumenstrom betrug 4 m³/h (Normbedingungen). Um das Druckverlustverhalten zu quantifizieren, wurde der Staudruck in einem 4 m langen Rohr mit einem Innendurchmesser von 21 mm in Abhängigkeit von der Schütthöhe vermessen. In einer Testreihe wurden die Formkörper der Beispiele 1 bis 3 sowie des Vergleichsbeispiels aus dem Vanadium-Phosphor-Mischoxid (VPO) verwendet. Eine andere Testreihe betraf die Beispiele 1a, 2a, 3a, und das Vergleichsbeispiel a, die entsprechend den Formkörpern der Beispiele 1 bis 3 sowie des Vergleichsbeispiels geformt waren, jedoch aus dem Al₂O₃/ SiO₂ Mischoxid bestanden.

In Fig. 4 ist der resultierende Staudruck der Testreihe der Beispiele 1 bis 3 sowie des Vergleichsbeispiels als Funktion der Schüttungshöhe aufgetragen. Es ist aus Fig. 4 ersichtlich, dass der Druckverlust bei Verwendung einer Schüttung des Beispiels 1, einer Schüttung des Beispiels 2 und einer Schüttung des Beispiels 3 deutlich geringer war als bei Verwendung einer Schüttung des Vergleichsbeispiels. Die Testreihe der Beispiele 1a, 2a, 3a, und des Vergleichsbeispiels a ist nicht in Fig. 4 gezeigt, da sie zur mit der Testreihe der Beispielen 1 bis 3 sowie des Vergleichsbeispiels identischen Ergebnissen führte. Es zeigte sich also, dass es keinen Unterschied im Staudruckverhalten gibt, wenn Formkörper aus VPO oder Al₂O₃ hergestellt werden. Die eventuellen Unterschiede in der Oberflächenrauhigkeit spielen keine oder nur eine untergeordnete Rolle.

Tabellen 2 und 3 zeigen die Ergebnisse der Performancemessungen bei zwei verschiedenen GHSVs. Die Länge der Schüttung betrug jeweils 5,5 m dem Reaktor mit einem Rohrinnendurchmesser von 21 mm. Als Reaktoreingangsdruck waren jeweils 2650 mbar (absolut) eingestellt. Für die Messungen der Tabelle 2 war eine GHSV von 1810 h⁻¹ und für die Messungen der Tabelle 3 war eine GHSV von 2015 h⁻¹ eingesetzt.

**Tabelle 2:**

| Länge der Schüttung: 5,5 m; Rohrdurchmesser: 21 mm; Reaktoreingangsdruck: 2650 mbar (absolut); GHSV: 1810 h⁻¹ | | | | |
|---|---|---|---|---|
| Bedingungen/Ergebnisse | Ringe Vergleichsbeispiel | Große Doppelalphas Beispiel 1 | Große Doppelalphas Beispiel 2 | Kleine Doppelalphas Beispiel 3 |
| Salzbadtemperatur, °C | 420 | 415 | 419 | 421 |
| Hot Spot Temperatur, °C | 457 | 443 | 453 | 449 |
| Umsatz, mol % | 85,5 | 86,5 | 85,0 | 85,5 |
| Selektivität, mol % | 68,5 | 67,1 | 68,9 | 71,0 |
| Druckverlust, mbar | 800 | 515 | 590 | 550 |
| MA Produktivität, g_{MA}/ (kg_{Kat}·h) | 115 | 128 | 118 | 147 |
| Produktivität/ Druckverlust, g_{MA}/ (kg_{Kat}·h·mbar) | 0,144 | 0,249 | 0,198 | 0,267 |

**Tabelle 3:**

| Länge der Schüttung: 5,5 m; Rohrdurchmesser: 21 mm, Reaktoreingangsdruck: 2650 mbar (absolut); GHSV: 2015 h⁻¹ | | | | |
|---|---|---|---|---|
| Bedingungen/Ergebnisse | Ringe Vergleichsbeispiel | Große Doppelalphas Beispiel 1 | Große Doppelalphas Beispiel 2 | Kleine Doppelalphas Beispiel 3 |
| Salzbadtemperatur, °C | 421 | 416 | 419 | 423 |
| Hot Spot Temperatur, °C | 453 | 443 | 448 | 445 |
| Umsatz, mol % | 83 | 82 | 82 | 82 |
| Selektivität, mol % | 69,9 | 67,9 | 69,4 | 71,3 |
| Druckverlust, mbar | 990 | 650 | 740 | 690 |
| MA Produktivität, g_{MA}/ (kg_{Kat}·h) | 124 | 137 | 126 | 157 |
| Produktivität/ Druckverlust, g_{MA}/ (kg_{Kat}·h·mbar) | 0,125 | 0,211 | 0,170 | 0,228 |

Wie aus den Tabellen 2 und 3 ersichtlich ist, werden bei vergleichbaren Umsätzen an Butan und vergleichbaren Salzbadtemperaturen höhere Produktivitäten mit den Katalysatorformkörpern 100, 200 und 300 der Beispiele 1 bis 3 erreicht als mit den Ringgeometrien des Vergleichsbeispiels. Die durch die Katalysatorformen der Beispiele 1 bis 3 erreichte Verbesserung gegenüber dem Vergleichsbeispiel wird besonders deutlich gezeigt anhand des Quotient aus Produktivität und Druckverlust (= Produktivität pro Energieaufwand). Hierbei ergibt sich für den Quotienten folgende Reihenfolge: Beispiel 3 > Beispiel 1 > Beispiel 2 > Vergleichsbeispiel.

Weiterhin wird aus den Tabellen 2 und 3 deutlich, dass die Maximaltemperatur im Katalysatorbett (Hot Spot Temperatur) mit den Katalysatorformkörpern 100, 200 und 300 der Beispiele 1 bis 3 gegenüber dem Vergleichsbeispiel geringer ist. Dies ist darauf zurückzuführen, dass mit den Katalysatorformkörpern 100, 200 und 300 der Beispiele 1 bis 3 eine verbesserte Wärmeabfuhr und eine verbesserte Wärmeverteilung im Katalysatorbett gegenüber dem Vergleichsbespiel erreicht wird. Diese Effekte werden in den Tabellen 2 und 3 durch die Temperaturunterschiede zwischen dem Salzbad und der Hot Spot Temperatur der Beispiele 1 bis 3 dokumentiert, die im Vergleich zum Unterschied der Salzbadtemperatur zur Hot Spot Temperatur des Vergleichsbeispiels geringer sind.

**Tabelle 4:**

| Charakteristische Daten der Doppelalphas im Vergleich zu Charakteristika verschiedener Ringe | | | |
|---|---|---|---|
| Formkörper | Schüttdichte¹) kg/l | Tablettendichte²) kg/l | Mittlere Seitendruck-festigkeit, N |
| Doppelalphas (100) Beispiel 1 | 0,62 | 1,70 | 31, 223³) |
| Doppelalphas (200) Beispiel 2 | 0,69 | 1,71 | 34, 230³) |
| Doppelalphas (300) Beispiel 3 | 0,56 | 1,58 | 40, 106³) |
| Ringe Vergleichsbeispiel (ø = 5,6 mm) | 0,76 | 1,75 | 24 |

| | | | |
|---|---|---|---|
| ¹⁾ gemessen im Rohr mit Lange 1 m und Innendurchmesser 21 mm ²⁾ gemessen über Hg-Porosimetrie ³⁾ erster Bereich wenig stabile Seite, zweiter Bereich stabile Seite | | | |

In Tabelle 4 sind zusätzliche charakteristische Daten der Katalysatorformkörper 100, 200 und 300 der Beispiele 1 bis 3 im Vergleich zu Daten von Ringen des Vergleichsbeispiels aufgeführt. Die Schüttdichte wurde im Rohr mit einer Länge von 1 m und einem Innendurchmesser von 21 mm angelehnt an DIN ISO 697 bestimmt. Unterschiede des verwendeten Messverfahrens zum Verfahren nach DIN ISO 697 waren das Messvolumen, erhalten durch die Länge von 1 m des Rohres und dem Innendurchmesser von 21 mm, und die Verwendung von Formkörpern statt Pulver. Die Tablettendichte wurde mittels Hg-Porosimetrie (Porotec, Pascal440 Series) gemessen, gemäß DIN 66133. Die Seitendruckfestigkeit wurde mit einem Tablettentester durchgeführt (Firma Phramatron, Dr. Schleuniger, Model 6D), gemäß ASTM D4179-88a. Es zeigt sich, dass die sogenannten Doppelalphas im Vergleich zu Ringen neben einem niedrigerem Druckverlustverhalten im Festbett eine geringere Schüttdichte, eine geringere Tablettendichte und eine höhere mechanische Stabilität, in der Raumrichtung der längeren Seite der Außen-Grundfläche sogar 5 - 10 mal höher, aufweisen.

## Patentansprüche

1. Katalysatorformkörper zur katalytischen Umsetzung von organischen und anorganischen Verbindungen in Festbettreaktoren,
wobei der Katalysatorformkörper (100; 200; 300; 400) als Zylinder mit einer Außen-Grundfläche (150), einer Zylinderfläche (152), einer Zylinderachse (154) und mindestens einer durchgehenden, parallel zur Zylinderachse verlaufenden Öffnung (160) ausgebildet ist und die Außen-Grundfläche des Zylinders mindestens vier Loben (110, 120, 130, 140; 210; 220, 230, 240; 310, 320, 330, 340; 410, 420, 430, 440) aufweist,
wobei ein den Katalysatorformkörper umschließender geometrischer Grundkörper ein Prisma (10) ist, das eine Prismengrundfläche mit einer Länge und einer Breite aufweist, wobei die Länge größer ist als die Breite,
wobei die Loben von Prismenecken der Prismengrundfläche umschlossen sind.

2. Katalysatorformkörper nach einem der vorstehenden Ansprüche, wobei zwischen zwei benachbarten Loben eine Vertiefung (172) in der Zylinderfläche vorgesehen ist, und/oder zwischen zwei benachbarten Loben eine Erhebung (170) in der Zylinderfläche vorgesehen ist.

3. Katalysatorformkörper nach einem der vorstehenden Ansprüche,
wobei der Katalysatorformkörper eine parallel zur Zylinderachse verlaufende Öffnung (160) und/oder vier Loben aufweist; und/oder
wobei der Katalysatorformkörper zwei entgegengesetzt angeordnete Vertiefungen und/oder zwei entgegengesetzt angeordnete Erhebungen aufweist.

4. Katalysatorformkörper nach einem der vorstehenden Ansprüche,
wobei der Katalysatorformkörper ein Verhältnis der geometrischen Oberfläche des Katalysatorformkörpers zum Volumen des Katalysatorformkörpers von 1 bis 1,8 mm⁻¹ besitzt; und/oder
wobei mindestens ein Element ausgewählt aus den Loben, der Vertiefung, den Vertiefungen, der Erhebung und den Erhebungen abgerundet ist.

5. Katalysatorformkörper nach einem der Ansprüche 1 bis 4, wobei das Prisma (10) ein Quader ist; und/oder
wobei das Verhältnis des Volumens des Katalysatorformkörpers zum Volumen des umgebenden Quaders größer als 80 % ohne Berücksichtigung der mindestens einen Öffnung und größer 70 % unter Berücksichtigung der mindestens einen Öffnung ist; und/oder
wobei zwei Loben die Länge der Prismengrundfläche definieren und/oder zwei Loben die Breite der Prismengrundfläche definieren.

6. Katalysatorformkörper nach einem der Ansprüche 1 bis 5, wobei die Erhebung oder Erhebungen zwischen Loben vorgesehen ist/sind, die die Länge definieren, und/oder die Vertiefung oder Vertiefungen zwischen Ecken vorgesehen ist/sind, die die Breite definieren.

7. Katalysatorformkörper nach einem der Ansprüche 4 bis 6, wobei die Loben abgerundet sind, jeweils einen Schwerpunkt (180) besitzen, und/oder mindestens zwei der Loben voneinander verschieden sind; und/oder
wobei der Schwerpunkt jeweils von der Zylinderachse 1,5 bis 5 mm, bevorzugt 2,5 bis 4 mm, entfernt ist; und/oder wobei die abgerundeten Loben einen Durchmesser der Rundung im Bereich von 1 bis 2 mm besitzen; und/oder wobei der Radius des von den abgerundeten Loben jeweils gebildeten Kreisbogens im Bereich 0,5 bis 2,5 mm liegt; und/oder
wobei der Radius des von den abgerundeten Erhebungen jeweils gebildeten Kreisbogens im Bereich 2 bis 3,5 mm liegt; und/oder
wobei der Radius des von den abgerundeten Vertiefungen jeweils gebildeten Kreisbogens im Bereich 1 bis 9 mm liegt.

8. Katalysatorformkörper nach Anspruch 7, wobei die Loben derart in der Außen-Grundfläche angeordnet sind, dass der Winkel zwischen der Senkrechten auf eine Gerade durch die Schwerpunkte zweier Ecken, die die Breite der Prismengrundfläche definieren, und einer Geraden durch den Schwerpunkt dieser Ecken und durch die Zylinderachse 10° bis 75°, bevorzugt 25°bis 60°, beträgt.

9. Katalysatorformkörper nach einem der vorstehenden Ansprüche, wobei die Abmessung oder Höhe des Katalysatorformkörpers parallel zur Zylinderachse 2 bis 20 mm, bevorzugt 3 bis 6 mm beträgt; und/oder
wobei die Länge der Prismengrundfläche 2 bis 10 mm, bevorzugt 4 bis 8 mm beträgt.

10. Katalysatorformkörper nach einem der Ansprüche 2 bis 9, wobei der Außendurchmesser des Zylinders oder der Abstand zwischen zwei gegenüberliegenden Vertiefungen 3 bis 10 mm, bevorzugt 5 bis 8 mm, beträgt; und/oder
wobei der Durchmesser der Öffnung 0,5 bis 4 mm, bevorzugt 1 bis 3 mm beträgt.

11. Katalysatorformkörper nach einem der vorstehenden Ansprüche, wobei die Schüttdichte des Katalysatorformkörpers kleiner als 0,75 kg/l ist; und/oder
wobei eine mittlere unidirektionale Seitendruckfestigkeit größer als 28 N und/oder eine andere mittlere unidirektionale Seitendruckfestigkeit größer als 70 N ist.

12. Katalysatorformkörper nach einem der vorstehenden Ansprüche, umfassend
mindestens ein Element ausgewählt aus gemischten Oxiden des Vanadiums und des Phosphors, ein oder mehrere Metalle der Nebengruppen des Periodensystems, ein Metalloxid oder ein Metallmischoxid von Metallen der Nebengruppen des Periodensystems, und ein oder mehrere Edelmetalle.

13. Katalysatorformkörper nach einem der vorstehenden Ansprüche, umfassend mindestens ein Element ausgewählt aus Aluminiumoxid, Siliziumdioxid, Aluminiumsilikat, Zirkondioxid, und Titandioxid.

14. Verwendung eines Katalysatorformkörpers nach einem der Ansprüche 1 bis 12 für eine Partialoxidationsreaktion, für eine Partialoxidationsreaktion eines oder mehrerer Kohlenwasserstoffe, zur Herstellung von Maleinsäureanhydrid aus Kohlenwasserstoff oder n-Butan, zur Herstellung von Vinylacetatmonomer durch Oxidation von Ethen in Gegenwart von Essigsäure, oder zur Oxidation von Propen oder Propan zu Acrolein und/oder Acrylsäure.

## Claims

1. Shaped catalyst body for the catalytic reaction of organic and inorganic compounds in fixed-bed reactors,
wherein the shaped catalyst body (100; 200; 300; 400) is configured as cylinder having an outer base area (150), a cylindrical surface (152), a cylinder axis (154) and at least one continuous opening (160) running parallel to the cylinder axis and the outer base area of the cylinder has at least four lobes (110, 120, 130, 140; 210; 220, 230, 240; 310, 320, 330, 340; 410, 420, 430, 440),
wherein a geometric main body enclosing the shaped catalyst body is a prism (10) which has a prism base area having a length and a width, wherein the length is greater than the width,
wherein the lobes are enclosed by prism corners of the prism base area.

2. Shaped catalyst body according to any of the preceding claims, wherein a depression (172) in the cylindrical surface is provided between two adjacent lobes and/or a raised region (170) in the cylindrical surface is provided between two adjacent lobes.

3. Shaped catalyst body according to either of the preceding claims,
wherein the shaped catalyst body has an opening (160) running parallel to the cylinder axis and/or four lobes; and/or
wherein the shaped catalyst body has two depressions arranged opposite one another and/or two raised regions arranged opposite one another.

4. Shaped catalyst body according to any of the preceding claims,
wherein the shaped catalyst body has a ratio of the geometric surface area of the shaped catalyst body to the volume of the shaped catalyst body of from 1 to 1.8 mm⁻¹; and/or
wherein at least one element, selected from among the lobes, the depression, the depressions, the raised region and the raised regions, is rounded.

5. Shaped catalyst body according to any of Claims 1 to 4,
wherein the prism (10) is a cuboid; and/or
wherein the ratio of the volume of the shaped catalyst body to the volume of the surrounding cuboid is greater than 80% without taking into account the at least one opening and is greater than 70% taking into account the at least one opening; and/or
wherein two lobes define the length of the prism base area and/or two lobes define the width of the prism base area.

6. Shaped catalyst body according to any of Claims 1 to 5, wherein the raised region or raised regions is/are provided between lobes, which define the length, and/or the depression or depressions is/are provided between corners, which define the width.

7. Shaped catalyst body according to any of Claims 4 to 6, wherein the lobes are rounded, each have a centre of gravity (180), and/or at least two of the lobes are different from one another; and/or wherein the centre of gravity is in each case at a distance of from 1.5 to 5 mm, preferably from 2.5 to 4 mm, from the cylinder axis; and/or
wherein the rounded lobes have a diameter of the rounding in the range from 1 to 2 mm; and/or wherein the radius of the arc formed in each case by the rounded lobes is in the range from 0.5 to 2.5 mm; and/or
wherein the radius of the arc formed in each case by the rounded raised regions is in the range from 2 to 3.5 mm; and/or
wherein the radius of the arc formed in each case by the rounded depressions is in the range from 1 to 9 mm.

8. Shaped catalyst body according to Claim 7, wherein the lobes are arranged in the outer base area in such a way that the angle between the normals to a straight line through the centres of gravity of two corners, which define the width of the prism base area, and a straight line through the centre of gravity of these corners and through the cylinder axis is from 10° to 75°, preferably from 25° to 60°.

9. Shaped catalyst body according to any of the preceding claims, wherein the dimension or height of the shaped catalyst body parallel to the cylinder axis is from 2 to 20 mm, preferably from 3 to 6 mm; and/or wherein the length of the prism base area is from 2 to 10 mm, preferably from 4 to 8 mm.

10. Shaped catalyst body according to any of Claims 2 to 9, wherein the external diameter of the cylinder or the distance between two depressions located opposite one another is from 3 to 10 mm, preferably from 5 to 8 mm; and/or
wherein the diameter of the opening is from 0.5 to 4 mm, preferably from 1 to 3 mm.

11. Shaped catalyst body according to any of the preceding claims, wherein the bulk density of the shaped catalyst body is less than 0.75 kg/l; and/or wherein one average unidirectional lateral compressive strength is greater than 28 N and/or another average unidirectional lateral compressive strength is greater than 70 N.

12. Shaped catalyst body according to any of the preceding claims, comprising
at least one element selected from among mixed oxides of vanadium and phosphorus, one or more metals of the transition groups of the Periodic Table, a metal oxide or a mixed metal oxide of metals of the transition groups of the Periodic Table and one or more noble metals.

13. Shaped catalyst body according to any of the preceding claims, comprising at least one element selected from among aluminium oxide, silicon dioxide, aluminium silicate, zirconium dioxide and titanium dioxide.

14. Use of a shaped catalyst body according to any of Claims 1 to 12 for a partial oxidation reaction, for a partial oxidation reaction of one of more hydrocarbons, for the production of maleic anhydride from hydrocarbon or n-butane, for the production of vinyl acetate monomer by oxidation of ethene in the presence of acetic acid or for the oxidation of propene or propane to give acrolein and/or acrylic acid.

## Revendications

1. Corps moulé de catalyseur pour la conversion catalytique de composés organiques et inorganiques dans des réacteurs à lit fixe,
dans lequel le corps moulé de catalyseur (100; 200; 300; 400) est réalisé sous forme de cylindre avec une face de base extérieure (150), une face de cylindre (152), un axe de cylindre (154) et au moins une ouverture continue (160) s'étendant parallèlement à l'axe de cylindre et la face de base extérieure du cylindre présente au moins quatre lobes (110, 120, 130, 140; 210, 220, 230, 240; 310, 320, 330, 340; 410, 420, 430, 440),
dans lequel un corps de base géométrique enveloppant le corps moulé de catalyseur est un prisme (10), qui présente une face de base de prisme avec une longueur et une largeur, dans lequel la longueur est plus grande que la largeur,
dans lequel les lobes sont enveloppés par des coins de prisme de la face de base de prisme.

2. Corps moulé de catalyseur selon l'une quelconque des revendications précédentes, dans lequel il est prévu entre deux lobes voisins un creux (172) dans la face de cylindre, et/ou il est prévu entre deux lobes voisins une surélévation (170) dans la face de cylindre.

3. Corps moulé de catalyseur selon l'une quelconque des revendications précédentes,
dans lequel le corps moulé de catalyseur présente une ouverture (160) s'étendant parallèlement à l'axe de cylindre et/ou quatre lobes; et/ou
dans lequel le corps moulé de catalyseur présente deux creux disposés l'un en face de l'autre et/ou deux surélévations disposées l'une en face de l'autre.

4. Corps moulé de catalyseur selon l'une quelconque des revendications précédentes,
dans lequel le corps moulé de catalyseur possède un rapport de la surface géométrique du corps moulé de catalyseur au volume du corps moulé de catalyseur de 1 à 1,8 mm⁻¹; et/ou
dans lequel au moins un élément choisi parmi les lobes, le creux, les creux, la surélévation et les surélévations est arrondi.

5. Corps moulé de catalyseur selon l'une quelconque des revendications 1 à 4,
dans lequel le prisme (10) est un parallélépipède; et/ou dans lequel le rapport du volume du corps moulé de catalyseur au volume du parallélépipède d'enveloppe est supérieur à 80 % sans tenir compte de ladite au moins une ouverture et supérieur à 70 % en tenant compte de ladite au moins une ouverture; et/ou
dans lequel deux lobes définissent la longueur de la face de base de prisme et/ou deux lobes définissent la largeur de la face de base de prisme.

6. Corps moulé de catalyseur selon l'une quelconque des revendications 1 à 5, dans lequel la surélévation ou les surélévations est/sont prévue(s) entre des lobes, qui définissent la longueur, et/ou le creux ou les creux est/sont prévu(s) entre des lobes, qui définissent la largeur.

7. Corps moulé de catalyseur selon l'une quelconque des revendications 4 à 6,
dans lequel les lobes sont arrondis, présentent chacun un centre de gravité (180), et/ou au moins deux des lobes sont différents l'un de l'autre; et/ou
dans lequel le centre de gravité est respectivement espacé de l'axe de cylindre de 1,5 à 5 mm, de préférence de 2,5 à 4 mm; et/ou
dans lequel les lobes arrondis présentent un diamètre de l'arrondi situé dans la plage de 1 à 2 mm; et/ou dans lequel le rayon de l'arc de cercle respectivement formé par les lobes arrondis se situe dans la plage de 0,5 à 2,5 mm; et/ou
dans lequel le rayon de l'arc de cercle respectivement formé par les surélévations arrondies se situe dans la plage de 2 à 3,5 mm; et/ou
dans lequel le rayon de l'arc de cercle respectivement formé par les creux arrondis se situe dans la plage de 1 à 9 mm.

8. Corps moulé de catalyseur selon la revendication 7,
dans lequel les lobes sont disposés dans la face de base extérieure de telle manière que l'angle entre les perpendiculaires à une droite passant par les centres de gravité de deux coins, qui définissent la largeur de la face de base de prisme, et une droite passant par le centre de gravité de ces coins et par l'axe de cylindre vaut 10° à 75°, de préférence 25° à 60°.

9. Corps moulé de catalyseur selon l'une quelconque des revendications précédentes,
dans lequel la dimension ou la hauteur du corps moulé de catalyseur parallèlement à l'axe de cylindre vaut 2 à 20 mm, de préférence 3 à 6 mm; et/ou
dans lequel la longueur de la face de base de prisme vaut 2 à 10 mm, de préférence 4 à 8 mm.

10. Corps moulé de catalyseur selon l'une quelconque des revendications 2 à 9,
dans lequel le diamètre extérieur du cylindre ou la distance entre deux creux opposés vaut 3 à 10 mm, de préférence 5 à 8 mm; et/ou
dans lequel le diamètre de l'ouverture vaut 0,5 à 4 mm, de préférence 1 à 3 mm.

11. Corps moulé de catalyseur selon l'une quelconque des revendications précédentes,
dans lequel la masse volumique apparente du corps moulé de catalyseur est inférieure à 0,75 kg/l; et/ou
dans lequel une résistance à la compression latérale unidirectionnelle moyenne est supérieure à 28 N et/ou une autre résistance à la compression latérale unidirectionnelle moyenne est supérieure à 70 N.

12. Corps moulé de catalyseur selon l'une quelconque des revendications précédentes, comprenant au moins un élément choisi parmi des oxydes mixtes de vanadium et de phosphore, un métal ou plusieurs métaux des sous-groupes du système périodique, un oxyde métallique ou un oxyde métallique mixte de métaux des sous-groupes du système périodique, et un ou plusieurs métaux nobles.

13. Corps moulé de catalyseur selon l'une quelconque des revendications précédentes, comprenant au moins un élément choisi parmi l'oxyde d'aluminium, le dioxyde de silicium, le silicate d'aluminium, le dioxyde de zirconium et le dioxyde de titane.

14. Utilisation d'un corps moulé de catalyseur selon l'une quelconque des revendications 1 à 12 pour une réaction d'oxydation partielle, pour une réaction d'oxydation partielle d'un ou de plusieurs hydrocarbure(s), pour la production d'anhydride de l'acide maléique à partir d'hydrocarbure ou de n-butane, pour la production de monomère d'acétate de vinyle par oxydation d'éthène en présence d'acide acétique, ou pour l'oxydation de propène ou de propane en acroléine et/ou en acide acrylique.
